# FASCICULE DE BREVET EUROPEEN

(11) **EP 1 139 932 B1**
(45) Date de publication et mention de la délivrance du brevet: **19.07.2006**
(21) Numéro de dépôt: 99958282.8
(22) Date de dépôt: 09.12.1999
(51) Int. Cl.: A61F 2/44

(54) **PROTHESE DE DISQUE INTERVERTEBRAL A FROTTEMENTS REDUITS**
BANDSCHEIBENPROTHESE MIT VERMINDERTEM VERSCHLEISS
INTERVERTEBRAL DISC PROSTHESIS WITH REDUCED FRICTION

(30) Priorité: 11.12.1998 FR 9815669
(43) Date de publication de la demande: 10.10.2001
(73) Titulaire: Stryker Spine, 33610 Cestas (FR)
(72) Inventeur: GAUCHET, Fabien, 60800 Duvy (FR)
(74) Mandataire: Le Forestier, Eric
(86) Numéro de dépôt international: PCT/FR1999/003070
(87) Numéro de publication internationale: WO 2000/035382

(56) Documents cités:
- EP-A- 0 277 282
- EP-A- 0 642 775
- DE-A- 3 939 593
- FR-A- 2 723 841
- FR-A- 2 728 037

## Description

L'invention concerne les prothèses de disque intervertébral.

On connaît d'après le document EP-0 277 282 une telle prothèse comprenant deux plateaux destinés à venir en contact avec les plateaux vertébraux des vertèbres adjacentes au disque à remplacer, et un coussin interposé entre les plateaux et comprenant un corps compressible formant une enceinte pour un liquide. Toutefois, il peut survenir une usure entre le corps compressible et les plateaux, conduisant notamment à l'émission de particules solides et à leur dispersion dans le corps humain. De plus, bien que le comportement mécanique de cette prothèse approche de près celui d'un disque intervertébral naturel sain, on souhaite disposer d'une prothèse approchant différemment, voire d'encore plus près, le comportement d'un disque normal.

Un but de l'invention est de fournir une prothèse générant moins d'usure et ayant un comportement mécanique différent.

En vue de la réalisation de ce but, on prévoit selon l'invention une prothèse conforme à la revendication 1.

Ainsi, le corps est en contact avec au plus l'un des deux plateaux, voire aucun des deux. On réduit donc les frottements entre le corps et les plateaux, ainsi que l'usure et la génération de particules. Des modes de realisation ont enoncés aux revendications 2 à 4.

Avantageusement, le corps est immergé dans le fluide.

Avantageusement, le corps est mobile par rapport à chaque plateau.

Cette caractéristique réduit encore la probabilité d'un frottement entre le corps et les plateaux, le corps se disposant spontanément pour être sollicité le moins possible par les plateaux.

Avantageusement, le fluide est compressible.

Ainsi, le fluide non seulement renvoie sur toute la surface du corps et dans toutes les directions les sollicitations nées des mouvements relatifs des plateaux. Mais en outre, il encaisse lui-même une partie de ces sollicitations, l'autre partie étant encaissée par le corps compressible.

Avantageusement, le fluide a une résistance à la compression inférieure à celle du corps.

Avantageusement, le corps présente au moins une alvéole isolée de l'extérieur du corps.

La présence d'une ou plusieurs alvéoles influence le comportement mécanique du corps compressible en plus du choix du matériau et de ses dimensions.

Avantageusement, l'alvéole est remplie d'un deuxième fluide.

Avantageusement, le deuxième fluide a, lorsque la prothèse n'est pas sollicitée, une pression supérieure ou égale à celle du fluide de l'enceinte.

D'autres caractéristiques et avantages de l'invention apparaîtront encore dans la description suivante d'un mode préféré de réalisation donné à titre d'exemple non limitatif. Aux dessins annexés :
- la figure 1 est une vue en perspective avec arrachement d'un mode préféré de réalisation de la prothèse ;
- la figure 2 est une vue en coupe axiale de la prothèse de la figure 1 ;
- la figure 3 est une vue en perspective d'une variante de réalisation de la prothèse ; et
- la figure 4 est une vue en coupe axiale de la prothèse de la figure 3.

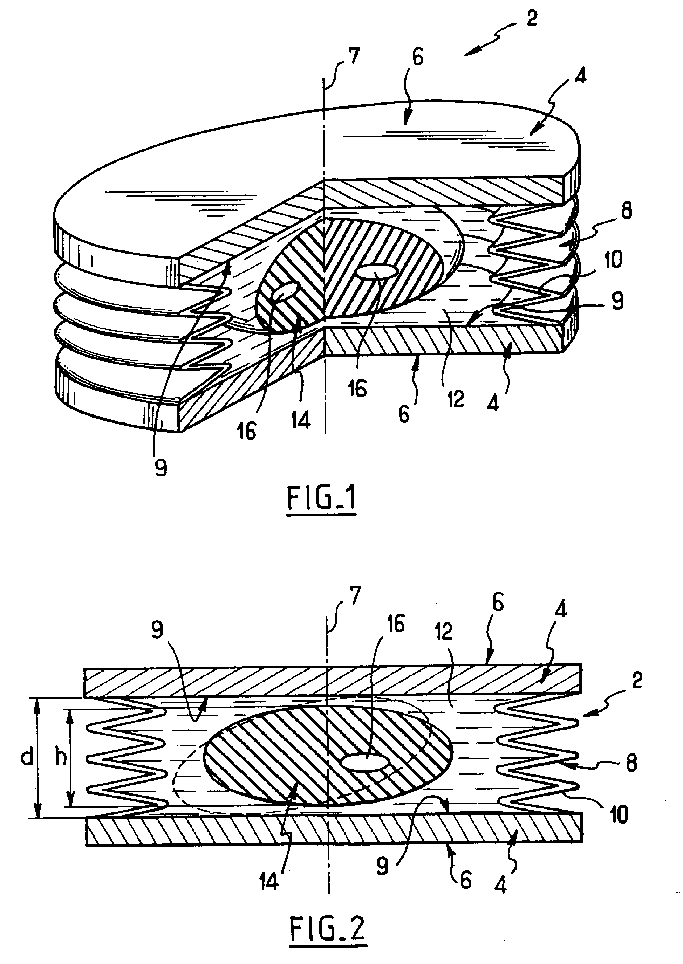

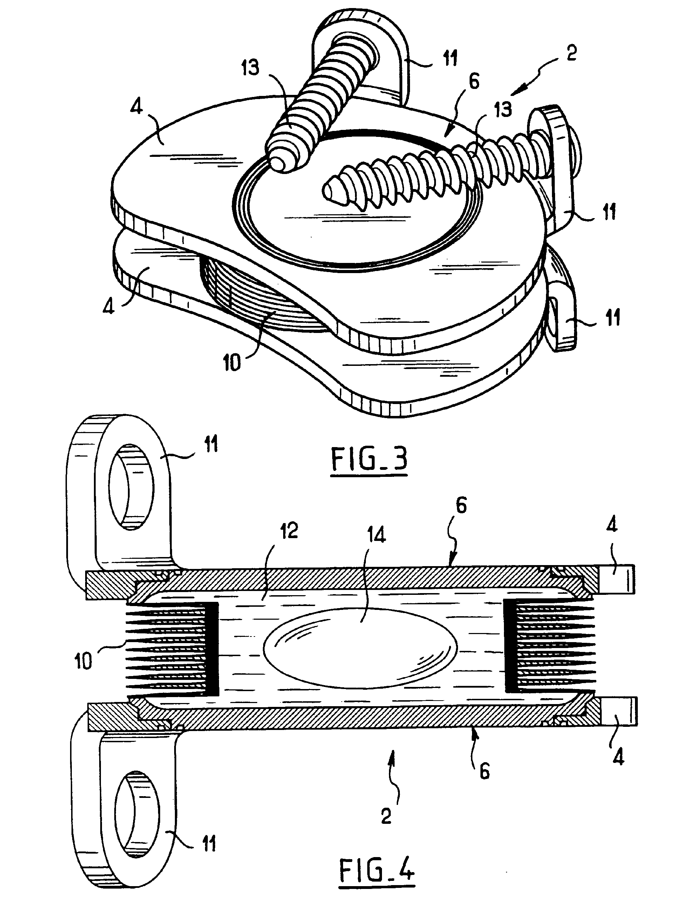

En référence aux figures 1 et 2, la prothèse de disque intervertébral 2 est ici de préférence destinée à la zone lombaire du rachis. Elle comporte deux plateaux 4 de forme générale plate qui ont été illustrés comme ayant en plan une forme de disque mais qui auront de préférence une forme en haricot à hile postérieur, comme illustré dans la variante des figures 3 et 4. Les deux plateaux 4 s'étendent parallèlement l'un à l'autre en regard l'un de l'autre. Sans que cela ait été illustré sur les figures et 1 et 2, mais comme on le voit sur la variante des figures 3 et 4, chaque plateau peut comprendre sur sa face externe 6 opposée à l'autre plateau au moins une patte de fixation 11 s'étendant en saillie de cette face et présentant un orifice pour le passage d'une vis à os 13 en vue de son ancrage dans la vertèbre en contact avec ce plateau. Chaque plateau 4 est ici en titane ou alliage de titane. Les plateaux ont un axe commun 7 constituant un axe principal de la prothèse perpendiculaire aux plateaux.

Pour réaliser un ancrage à court terme de la prothèse de disque 2 dans la colonne, on pourra ancrer les vis 13 dans le spondyle des vertèbres adjacentes au disque à remplacer. -

Toutefois, on pourra prévoir un ancrage dit à long terme où, en outre, les surfaces 6 des plateaux 4 en contact avec les vertèbres adjacentes sont recouvertes d'hydroxyapatite, ou de toute autre substance connue en soi pouvant stimuler la croissance osseuse. Avant recouvrement, lesdites surfaces 6 pourront être traitées pour obtenir un état de surface plus ou moins poreux, présentant des points d'ancrage pour le tissu osseux, pour assurer une meilleure interface avec ledit tissu osseux.

La prothèse comporte un coussin ou partie intermédiaire 8 interposé entre les plateaux. Le coussin 8 comporte une enceinte 10 constituée ici par un soufflet. Le soufflet a une forme symétrique de révolution autour de l'axe 7. Sa paroi présente de profil des ondulations permettant de faire varier la longueur du soufflet 10 suivant la direction axiale 7, sans que varie sensiblement la superficie de sa section transversalement à l'axe 7. En l'espèce, ce soufflet est réalisé en titane ou alliage de titane, de sorte qu'il présente une certaine rigidité axiale et forme un ressort de compression. Il peut également être déformé suivant une direction perpendiculaire à l'axe 7 ou subir une torsion autour de l'axe 7 ou d'un axe quelconque perpendiculaire à celui-ci. Le soufflet 10 présente à ses deux extrémités axiales des bords collés à des bords respectifs des plateaux 4 s'étendant, par exemple de façon illustrée aux figures 3 et 4, en saillie d'une face interne 9 des plateaux. Le collage est réalisé de façon étanche de sorte que le soufflet 10 définit avec les deux plateaux 4 une enceinte étanche.

Le soufflet 10 peut présenter dix convolutions, soit huit crêtes externes en plus des deux crêtes de fixation aux plateaux. Il a ici un diamètre externe d'environ 30 mm et un diamètre interne d'environ 17 mm. Sa hauteur, lorsque la prothèse est hors charge, vaut 10 mm. La paroi du soufflet peut être réalisée au moyen d'une, deux ou trois feuilles chacune de 0,1 mm d'épaisseur et dont la somme des épaisseurs forme l'épaisseur de la paroi. Le soufflet a ici en propre une raideur d'environ 1,6 N/mm.

L'enceinte définie par les plateaux 4 et le soufflet 10 renferme un fluide 12, ici compressible et biocompatible. Il s'agit ici d'un mélange d'un liquide et d'un gaz soluble partiellement dans le liquide. Le fluide 12 s'étend au contact direct des plateaux 4 et du soufflet 10. Le liquide pourra être de l'eau ou un sérum physiologique.

Le coussin 8 comporte également en plus du fluide 12 un corps compressible 14 qui pourra être réalisé dans un matériau élastiquement compressible tel qu'un élastomère, ou un matériau viscoélastique tel qu'un silicone, comme c'est le cas ici.

Le corps 14 a ici une forme d'ellipsoïde aplati suivant son axe autour duquel il présente une symétrie de révolution, cet axe étant confondu avec l'axe 7 sur les figures. On appellera « h » la plus petite hauteur hors tout du corps 14 mesurée suivant son axe de révolution. Cette distance ne sera pas nécessairement mesurée parallèlement à l'axe principal 7 de la prothèse puisque le corps 14 peut s'incliner de sorte que son axe de symétrie est incliné par rapport à l'axe principal 7 de la prothèse, comme illustré en traits pointillés sur la figure 2. Cette valeur h est instantanée. Elle est variable selon les circonstances puisque le corps 14 est compressible, notamment suivant son axe de symétrie. Le corps présente par ailleurs une autre dimension hors tout supérieure à h, et mesurée dans un plan perpendiculaire à son axe de symétrie. On désigne par « d » la distance instantanée séparant l'un de l'autre les centres des deux plateaux 4. Cette valeur elle aussi est instantanée, la distance entre les deux centres pouvant varier lorsque la prothèse est comprimée. Cette valeur est toujours mesurée suivant l'axe principal 7 de la prothèse.

La prothèse peut subir une compression suivant l'axe principal 7 tendant à rapprocher les deux plateaux 4 l'un de l'autre sans modifier leur inclinaison relative. Elle peut aussi subir une flexion autour d'un axe quelconque perpendiculaire à l'axe principal 7 et tendant à incliner les plateaux l'un par rapport à l'autre et donc à rapprocher une partie de leurs bords périphériques. Ces mouvements sont les principaux susceptibles de modifier la distance entre les plateaux : les mouvements de cisaillement tendant à déplacer relativement les plateaux 4 parallèlement à leur plan, et les mouvements de rotation relative des plateaux autour de l'axe 7 ne modifient pas la distance entre les plateaux de façon significative.

La prothèse est agencée de sorte que, quelles que soient les circonstances, notamment quelles que soient les sollicitations que subit la prothèse et la déformation qu'elle présente, le corps 14 peut toujours occuper spontanément une position dans laquelle il est en contact avec au plus un des plateaux 4, voire aucun des deux. Une telle position peut être une position inclinée dans laquelle l'axe de symétrie du corps 14 est incliné par rapport à l'axe principal 7 et/ou dans laquelle le corps est décentré par rapport à cet axe, comme illustré en traits pointillés sur la figue 2, ou encore une position dans laquelle l'axe de symétrie du corps est confondu avec l'axe principal 7 de la prothèse. Cette propriété résulte principalement du choix de la forme et des dimensions du corps 14, du volume de l'enceinte de fluide 12 et de la compressibilité du corps 14 et du fluide 12. En l'espèce, cette propriété du corps est obtenue d'autant plus facilement que le corps 14 est immergé dans le fluide 12 en étant totalement mobile par rapport à chacun des plateaux 4 sans aucun ancrage avec ceux-ci. L'homme du métier pourra sans difficulté réaliser des prothèses fonctionnant de cette façon. Cette propriété du corps 14 sera bien entendu valable lorsque la prothèse n'est pas sollicitée, c'est-à-dire avant la pose sur le patient. Elle sera également valable après la pose, en conditions d'utilisation. Par exemple, on pourra prévoir que cette propriété reste valable pour toute sollicitation en compression de la prothèse jusqu'à une intensité de 3 000 N, qui correspond à une intensité parfois supportée par un disque naturel sain, par exemple lorsque le patient porte une charge. Par sécurité, on pourra repousser cette limite jusqu'à une intensité de 5 000 N, intensité qui correspond à la limite de résistance des vertèbres en elles-mêmes.

Lorsque la plus petite dimension hors tout h du corps 14 est, au repos, seulement légèrement inférieure à la distance d séparant les centres des plateaux, comme c'est le cas sur les figures, il sera préférable de prévoir une importance possibilité de débattement latéral du corps 14 dans l'enceinte. Par exemple, la dimension de l'enceinte perpendiculairement à l'axe principal 7 sera comprise entre 1,3 et 1,5 fois la plus grande dimension hors tout du corps 14 suivant la même direction.

Le corps 14 comprend ici plusieurs alvéoles 16 fermées et isolées de l'extérieur du corps 14. Chaque alvéole renferme un fluide qui est ici un gaz ayant au repos de la prothèse une pression supérieure à la pression du fluide 12 de l'enceinte. Ces alvéoles 16 modifient le comportement du corps 14 en compression, notamment en réduisant localement sa compressibilité. Les alvéoles pourront être ou pas en communication les unes avec les autres.

La forme en ellipsoïde du corps 14 est particulièrement avantageuse puisqu'elle permet de donner au corps un grand volume et une grande surface de contact avec le fluide 12 de l'enceinte tout en lui donnant une faible dimension h et en permettant d'importants mouvements relatifs des plateaux aussi bien en compression qu'en flexion.

La variante des figures 3 et 4 comporte un coussin analogue à celui des figures 1 et 2.

Bien entendu, on pourra apporter à l'invention de nombreuses modifications sans sortir du cadre de celle-ci.

Le corps 14 pourra être fixé à l'un des plateaux 4, la prothèse étant agencée pour que l'autre plateau 4 ne puisse venir en contact avec le corps 14.

Le corps 14 pourra avoir différentes formes, par exemple une forme sphérique.

Le soufflet pourra avoir en section une forme elliptique.

Le fluide 12 pourra être un liquide.

Dans ce cas, on pourra choisir ce liquide ainsi que le matériau du corps 14 de sorte que le liquide ne mouille pas ce matériau bien qu'il puisse venir en contact avec lui. Une telle propriété implique qu'il faut fournir une certaine énergie pour produire ce contact, laquelle énergie est restituée lorsqu'on cesse de la fournir. Cet effet ressort est d'autant plus important que le corps est poreux. Lorsque les pores constituent de longs réseaux, la dissipation d'énergie produite lors de la circulation du liquide entrant ou sortant des pores produit un effet amortisseur se combinant à l'effet ressort pour donner une forme en hystérésis à la courbe illustrant l'intensité de la compression subie par la prothèse en fonction de la variation de la distance d.

## Revendications

1. Prothèse de disque intervertébral comprenant deux plateaux (4), et un coussin (8) interposé entre les plateaux et comportant une enceinte remplie de fluide (12) et un corps compressible (14), **caractérisée en ce que** le corps (14) est conformé de façon à pouvoir occuper une position dans laquelle il est en contact avec au plus un des plateaux (4) lorsque la prothèse subit une compression tendant à rapprocher les plateaux (4) l'un de l'autre.

2. Prothèse selon la revendication 1, **caractérisée en ce que** le corps (14) est cantonné pour occuper ladite position (12) a une résistance à la compression inférieure à celle du corps (14) .

3. Prothèse selon la revendication 2, **caractérisée en ce que** la compression a une intensité inférieure ou égale 5000N.

4. Prothèse conforme à l'une quelconque des revendications précédentes **caractérisée en ce que** le corps (14) est conformé de façon à pouvoir occuper ladite position lorsque la prothèse n'est pas sollicitée.

5. Prothèse selon l'une quelconque des revendications 1 à 4, **caractérisée en ce que** le corps (14) est immergé dans le fluide (12).

6. Prothèse selon l'une quelconque des revendications 1 à 5, **caractérisée en ce que** le corps (14) est mobile par rapport à chaque plateau (4).

7. Prothèse selon l'une quelconque des revendications 1 à 6, **caractérisée en ce que** le fluide (12) est compressible.

8. Prothèse selon l'une quelconque des revendications 1 à 7, **caractérisée en ce que** le fluide

9. Prothèse selon l'une quelconque des revendications 1 à 8, **caractérisée en ce que** le corps (14) présente au moins une alvéole (16) isolée de l'extérieur du corps.

10. Prothèse selon la revendication 9, **caractérisée en ce que** l'alvéole (16) est remplie d'un deuxième fluide.

11. Prothèse selon la revendication 10, **caractérisée en ce que** le deuxième fluide a, lorsque la prothèse n'est pas sollicitée, une pression supérieure ou égale à celle du fluide (12) de l'enceinte.

## Claims

1. Intervertebral disc prosthesis comprising two plates (4) , and a cushion (8) interposed between the plates and having a chamber filled with fluid (12) and a compressible body (14), **characterized in that** the body (14) is so shaped as to be able to take up a position in which it is in contact with at most one of the plates (4)

2. Prosthesis according to Claim 1, **characterized in that** the body (14) is shaped in order to take up said position when the prosthesis is subjected to compression tending to bring the plates (4) closer to each other.

3. Prosthesis according to Claim 2, **characterized in that** the compression has an intensity of less than or equal to 5000 N.

4. Prosthesis according to any one of the preceding claims, **characterized in that** the body (14) is so shaped as to be able to take up said position when the prosthesis is not stressed.

5. Prosthesis according to any one of Claims 1 to 4, **characterized in that** the body (14) is immersed in the fluid (12) .

6. Prosthesis according to any one of Claims 1 to 5, **characterized in that** the body (14) is movable relative to each plate (4).

7. Prosthesis according to any one of Claims 1 to 6, **characterized in that** the fluid (12) is compressible.

8. Prosthesis according to any one of Claims 1 to 7, **characterized in that** the fluid (12) has a resistance to compression less than that of the body (14) .

9. Prosthesis according to any one of Claims 1 to 8, **characterized in that** the body (14) has at least one cell (16) isolated from the outside of the body.

10. Prosthesis according to Claim 9, **characterized in that** the cell (16) is filled with a second fluid.

11. Prosthesis according to Claim 10, **characterized in that**, when the prosthesis is unstressed, the second fluid has a pressure greater than or equal to that of the fluid (12) in the chamber.

## Patentansprüche

1. Bandscheibenprothese, die zwei Platten (4) und ein Kissen (8) umfaßt, das zwischen den Platten sitzt und eine mit einem Fluid (12) gefüllte Einfassung und einen kompressiblen Korpus (14) aufweist, **dadurch gekennzeichnet, daß** der Korpus (14) so eingerichtet ist, daß er eine Position einnehmen kann, in der er mit höchstens einer der Platten (4) in Kontakt ist.

2. Prothese nach Anspruch 1, **dadurch gekennzeichnet, daß** der Korpus (14) so eingerichtet ist, daß er die besagte Position einnimmt, wenn die Prothese einer Kompression ausgesetzt ist, die versucht die Platten (4) einander anzunähern.

3. Prothese nach Anspruch 2, **dadurch gekennzeichnet, daß** die Kompression eine Stärke von weniger als oder gleich 5000 N hat.

4. Prothese nach einem der vorhergehenden Ansprüche, **dadurch gekennzeichnet, daß** der Korpus (14) so eingerichtet ist, daß er die besagte Position einnehmen kann, wenn die Prothese nicht beaufschlagt wird.

5. Prothese nach einem der Ansprüche 1 bis 4, **dadurch gekennzeichnet, daß** der Korpus (14) in das Fluid (12) eingetaucht ist.

6. Prothese nach einem der Ansprüche 1 bis 5, **dadurch gekennzeichnet, daß** der Korpus (14) in Bezug auf jede der Platten (4) beweglich ist.

7. Prothese nach einem der Ansprüche 1 bis 6, **dadurch gekennzeichnet, daß** das Fluid (12) kompressibel ist.

8. Prothese nach einem der Ansprüche 1 bis 7, **dadurch gekennzeichnet, daß** das Fluid (12) einen Widerstand gegen die Kompression hat, der geringer ist als der des Korpus (14).

9. Prothese nach einem der Ansprüche 1 bis 8, **dadurch gekennzeichnet, daß** der Korpus (14) wenigstens eine Zelle (16) hat, die vom Korpusäußeren isoliert ist.

10. Prothese nach Anspruch 9, **dadurch gekennzeichnet, daß** die Zelle (16) mit einer zweiten Flüssigkeit gefüllt ist.

11. Prothese nach Anspruch 10, **dadurch gekennzeichnet, daß** die zweite Flüssigkeit einen Druck hat, der, wenn die Prothese nicht beaufschlagt wird, größer oder gleich demjenigen des Fluids (12) der Einfassung ist.
